# FASCICULE DE BREVET EUROPEEN

(11) **EP 1 647 246 B1**
(45) Date de publication et mention de la délivrance du brevet: **07.03.2007**
(21) Numéro de dépôt: 05291495.9
(22) Date de dépôt: 11.07.2005
(51) Int. Cl.: A61F 5/01

(54) **Orthese dynamique polycentrique de l'articulation du genou**
Polyzentrische dynamische Orthese für das Kniegelenk
Polycentric dynamic orthesis for the knee joint

(30) Priorité: 28.07.2004 FR 0408305
(43) Date de publication de la demande: 19.04.2006
(73) Titulaire: Marc, Thierry, 34070 Montpellier (FR)
(72) Inventeur: Marc, Thierry, 34070 Montpellier (FR)
(74) Mandataire: Gosse, Michel

(56) Documents cités:
- US-A- 5 022 391

## Description

L'invention concerne une orthèse dynamique polycentrique de l'articulation du genou du genre comportant :
a) un dispositif articulé, disposé sur au moins l'une des faces externe ou interne du genou, adapté pour reproduire la cinématique du genou lors du mouvement de flexion et d'extension du membre inférieur concerné du patient ;
b) deux bras reliés à chaque dispositif articulé et disposés, pour le premier, sur au moins l'une des faces externe ou interne de la cuisse, selon un axe parallèle à celui du fémur, pour le second, sur au moins l'une des faces externe ou interne de la jambe, au niveau du mollet, selon un axe parallèle à celui du tibia ;
c) deux moyens de liaison, adaptés pour maintenir respectivement le ou les bras fémoraux sur la cuisse et le ou les bras tibiaux sur la jambe du patient.

L'articulation du genou est constituée de deux ligaments croisés (l'un antéro-externe et l'autre postéro-interne) et de deux ligaments latéraux (l'un externe et l'autre interne) dont les extrémités sont reliées respectivement à l'extrémité basse du fémur et à l'extrémité haute du tibia.

Les mouvements relatifs des surfaces articulaires fémorales et tibiales entre elles s'apparentent à un roulement-glissement durant les phases de flexion et

Lorsqu'une lésion partielle ou totale intervient sur au moins l'un des ligaments croisés ou latéraux de l'articulation d'un genou, la cinématique articulaire est perturbée. Le rapport entre le roulement et le glissement des surfaces articulaires n'est plus physiologique. Le mouvement anormal qui se produit alors va gêner la cicatrisation du ligament lésé et progressivement détériorer les structures articulaires (cartilage) et périarticulaires (capsule articulaire et ligaments latéraux).

Le traitement de ces lésions peut être soit orthopédique, soit chirurgical. Dans les deux cas une orthèse doit être mis en place pendant plusieurs semaines pour permettre une bonne cicatrisation.

Le but d'une orthèse dynamique du genou est de permettre l'usage de celui-ci, soit à titre provisoire dans l'attente de sa totale récupération, soit à plus long terme dans le cas de lésions irréversibles ou irréparables afin d'éviter, ou de différer, une intervention chirurgicale.

Le but principal poursuivi consiste à reproduire, le plus fidèlement possible, le mouvement naturel de l'articulation du genou.

### ARRIERE PLAN TECHNOLOGIQUE

Les dispositifs connus destinés à constituer une orthèse dynamique de l'articulation du genou sont généralement de deux types :
- les orthèses monocentriques (du type décrit dans le brevet FR-2477409) qui présentent l'inconvénient, en fonctionnement dynamique, de détériorer le genou, principalement au niveau des surfaces articulaires et des structures capsuloligamentaires, puisqu'elles ne reproduisent pas le mouvement naturel de l'articulation du genou ;
- les orthèses polycentriques (du type décrit dans le brevet US-5302169) qui présentent l'inconvénient, malgré l'ingéniosité des solutions mises en oeuvre pour y remédier, de ne pas reproduire fidèlement le mouvement naturel du genou et de provoquer des lésions ligamentaires et cartilagineuses.

Les orthèses de ce deuxième type sont principalement décrites dans les brevets US-4523585, EP-0267999 et US-4628916 qui mettent en oeuvre deux biellettes croisées et des moyens aptes à limiter leur course.

Elles cherchent toutes à reproduire la fonction des ligaments croisés en se basant sur des longueurs et des positions de points d'ancrage bien spécifiques qui ne reproduisent pas fidèlement la cinématique du genou.

Indépendamment des diverses conceptions et formes des biellettes décrites dans lesdits documents, tous les dispositifs en question ne répondent pas aux exigences de sécurité concernant le fonctionnement en ciseaux desdites biellettes qui peut être à l'origine de coupures

Pour y remédier, elles nécessitent des boîtier protecteurs allant à l'encontre de la simplicité de conception de l'ensemble.

L'état de la technique le plus proche est décrit dans le brevet US-A-5022391 dans lequel :
- le dispositif articulé est constitué de deux flasques discaux, en superposition, pourvus, pour le premier, de deux points articulés distincts et, pour le second, de deux autres points articulés distincts ;
- l'extrémité inférieure du bras fémoral est reliée à deux points articulés appartenant respectivement aux flasques discaux et l'extrémité supérieure du bras tibial est reliée aux deux autres points articulés appartenant respectivement auxdits flasques discaux ;
- les quatre points articulés sont disposés de manière à reproduire le diagramme des points de contact successifs physiologiques entre fémur et tibia ;
- les extrémités des bras fémoral et tibial sont placées entre lesdits deux flasques discaux.

Une telle conception apporte un plus sur le plan esthétique et plus particulièrement au niveau de la sécurité.

### RESUME DE L'INVENTION

L'invention vise donc à réaliser une orthèse dynamique polycentrique de l'articulation du genou qui reproduit quasi-fidèlement la trajectoire des surface articulaires.

L'orthèse, selon l'invention, se caractérise essentiellement en ce que les quatre points articulés sont disposés de manière à constituer, lorsque l'articulation est en extension, les sommets d'un quadrilatère déformable qui ont pour coordonnées, par rapport à des axes orthogonaux X et Y, (0,Y1) pour le premier (1a), (X2,Y1) pour le second (2a), (-X1,0) pour le troisième (2b) et (X3,Y3) pour le quatrième (1b), avec X2 supérieur à Y1, X3 supérieur à X2 et Y3 inférieur à Y1.

Selon des particularités de réalisation de l'invention, la valeur de Y3 est comprise entre 40 et 60% de celle de Y1, la valeur de X3 est comprise entre 140 et 160% de celle de X2 et la valeur de X1 est comprise entre 1 et 20% de celle de X2.

Selon d'autres particularités de réalisation de l'invention :
- les flasques discaux sont en total recouvrement lorsque l'articulation du genou est en extension et comportent des butées adaptées pour limiter leur course en extension et en flexion ainsi que des moyens d'immobilisation totale.

Les fonctions essentielles assurées par ladite prothèse sont :
- stabiliser un genou instable ;
- assurer une immobilisation limitée après un traumatisme ;
- empêcher une nouvelle blessure lors du retour à des activités normales,
- respecter les normes de sécurité ;
- être esthétique, facile à nettoyer, la plus légère possible ;
- laisser la peau respirer ;
- s'adapter à la morphologie de la personne.

### PRESENTATION DES FIGURES

Les caractéristiques et les avantages de l'invention vont apparaître plus clairement à la lecture de la description détaillée qui suit d'au moins un mode de réalisation préféré de celle-ci donné à titre d'exemple non limitatif et représenté aux dessins annexés.

Sur ces dessins :
- la figure 1 est une vue de principe frontale d'une orthèse selon l'invention en extension ;
- la figure 2 est une vue de principe frontale de l'orthèse selon la figure 1 dans une position de flexion intermédiaire comprise entre 0° et 90° ;
- la figure 3 est une vue de principe frontale de l'orthèse selon la figure 2 dans une position de flexion à 90° ;

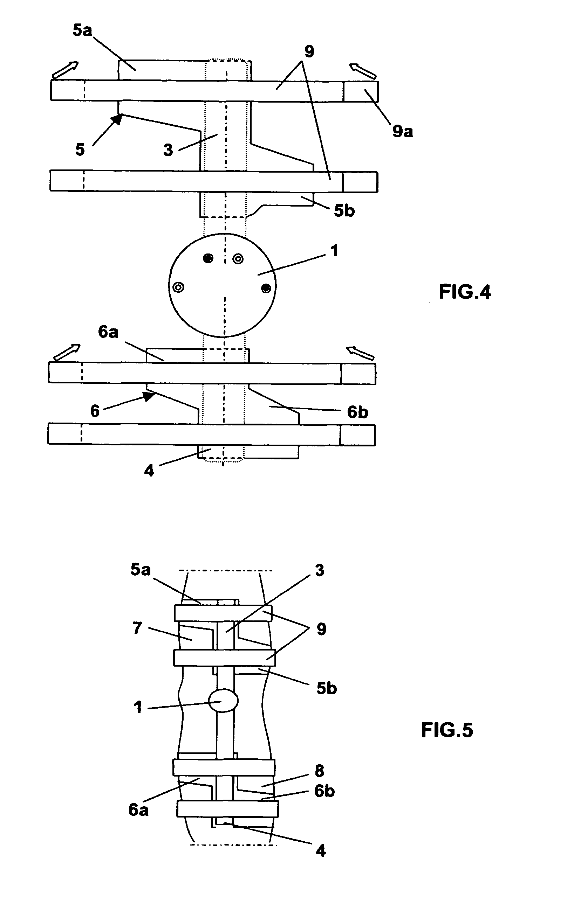

- la figure 4 est une vue de détail frontale d'une orthèse selon l'invention en extension ;
- la figure 5 est une vue frontale de l'orthèse de la figure 4 positionnée sur l'une des faces externe ou interne de la jambe du patient ;
- la figure 6 est un graphique donnant la position des points d'ancrage dans la solution consistant à reproduire au plus près la trajectoire des surface articulaires ;
- la figure 7 est un graphique donnant la trajectoire (C2,C3) de la position des points d'ancrage par rapport à la trajectoire physiologique (C1).

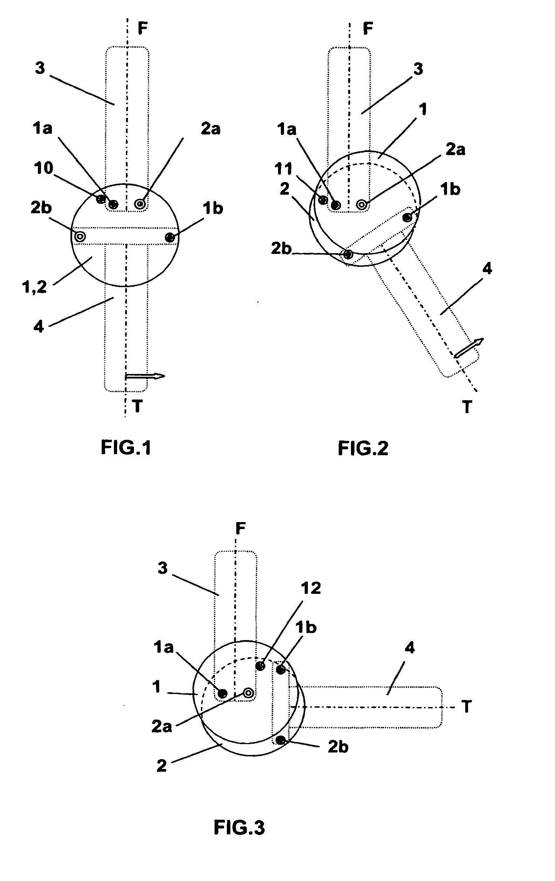

Pour une meilleure compréhension des figures 1 à 3, les bras fémoral et tibial sont apparents même pour les parties comprises entre les deux disques et le bras fémoral demeure vertical pendant les mouvements du genou.

### DESCRIPTION DETAILLEE DE L'INVENTION

L'orthèse dynamique polycentrique de l'articulation du genou (représentée aux figures) est du genre comportant essentiellement :
a) un dispositif articulé (1,2), disposé sur au moins l'une des faces externe ou interne du genou, adapté pour reproduire la cinématique du genou lors du mouvement de flexion et d'extension du membre inférieur concerné du patient ;
b) deux bras (3) et (4), reliés à chaque dispositif articulé (1,2), disposés, pour le premier, sur au moins l'une des faces externe ou interne de la cuisse (7), selon un axe parallèle à celui (F) du fémur, pour le second, sur au moins l'une des faces externe ou interne de la jambe (8), au niveau du mollet, selon un axe parallèle à celui (T) du tibia ;
c) deux moyens de liaison (5) et (6), adaptés pour maintenir respectivement le ou les bras fémoraux (3) sur la cuisse (7) et le ou les bras tibiaux (4) sur la jambe (8) du patient.

Selon des particularités de réalisation de ladite orthèse :
- le dispositif articulé est constitué de deux flasques discaux (1) et (2), en superposition, pourvus, pour le premier, de deux points articulés distincts (1a) et (1b) et, pour le second, de deux autres points articulés (2a) et (2b) ; en ce que l'extrémité inférieure du bras fémoral (3) est reliée à deux points articulés (1a) et (2a) appartenant respectivement aux flasques discaux (1) et (2) et l'extrémité supérieure du bras tibial (4) est reliée aux deux autres points articulés (1b) et (2b) appartenant respectivement auxdits flasques discaux (1) et (2) ;
   en ce que les quatre points articulés (1a), (1b), (2a) et (2b) sont disposés de manière à reproduire le diagramme des points de contact successifs physiologiques entre fémur et tibia ;
- les extrémités des bras fémoral et tibial sont placées entre lesdits deux flasques discaux (1) et (2).

Lesdits flasques discaux peuvent être réalisés en métal, en matière plastique rigide, en matériau composite ou en tout autre matériau.

Lesdits bras peuvent être également réalisés en métal, en matière plastique rigide, en matériau composite ou en tout autre matériau.

Selon les caractéristiques de base de l'invention, les quatre points articulés (1a), (1b), (2a) et (2b) sont disposés de manière à constituer, lorsque l'articulation est en extension, les sommets d'un quadrilatère déformable qui ont pour coordonnées, par rapport à des axes orthogonaux X et Y, (0,Y1) pour le premier (1 a), (X2,Y1) pour le second (2a), (-X1,0) pour le troisième (2b) et (X3,Y3) pour le quatrième (1b).

L'étude expérimentale (calculs de dimensionnement et modélisation) de la position de ces différents points pour approcher au mieux la cinématique physiologique du genou a donné pour résultats :
- X2 supérieur à Y1, X3 supérieur à X2 et Y3 inférieur à Y1;
- valeur de Y3 comprise entre 40 et 60% de celle de Y1, valeur de X3 comprise entre 140 et 160% de celle de X2 et valeur de X1 comprise entre 1 et 20% de celle de X2.

La courbe (C1) correspond à la trajectoire physiologique (surfaces articulaires).

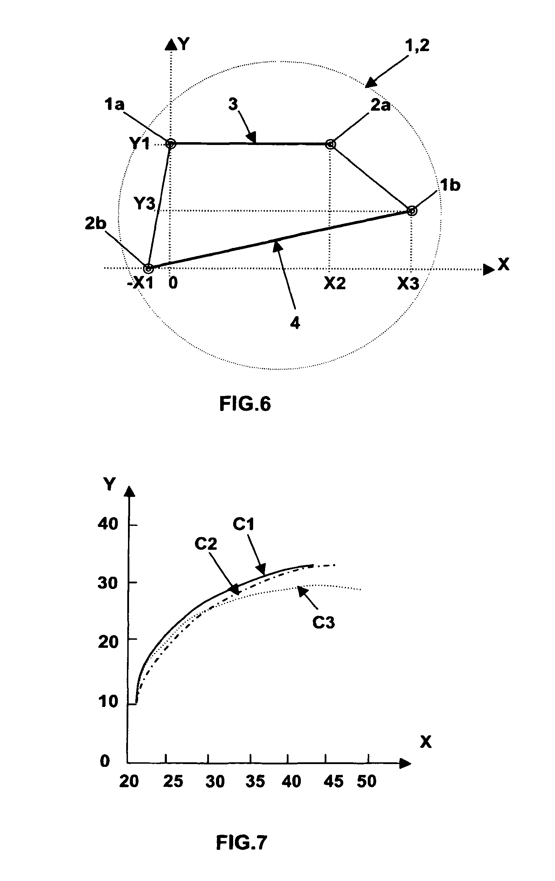

La courbe (C2) correspond à la trajectoire donnée par un positionnement des points d'ancrage comme indiqué à la figure 6.

La courbe (C3) correspond à la trajectoire donnée par un positionnement des points d'ancrage comme indiqué à la figure 6 excepté pour le point (1b) ou Y3 = 0.

Selon des particularités de réalisation de l'invention :
- le moyen de liaison (5), adapté pour maintenir le ou les bras fémoraux (3) sur les faces externe et ou interne de la cuisse (7) du patient, est constitué d'un premier élément (5a), apte à se plaquer sur la partie antérieure de la cuisse et d'un deuxième élément (5b) apte à se plaquer sur la partie postérieure de ladite cuisse ;
- le moyen de liaison (6), adapté pour maintenir le ou les bras tibiaux (4) sur la jambe (8) du patient, est constitué d'un premier élément (6a) apte à se plaquer sur la partie antérieure de celle-ci et d'un deuxième élément (6b) apte à se plaquer sur sa partie postérieure, au niveau du mollet ;
- chacun des éléments (5a), (5b), (6a) et (6b) constitutifs des moyens de liaison (5) et (6) est associé à une lanière (9) apte à entourer respectivement la cuisse ou le mollet et munie à ses extrémités d'un moyen de fermeture (9a) du type auto-agrippant ;
- les éléments (5a), (5b), (6a) et (6b) constitutifs des moyens de liaison (5) et (6) sont réalisés au moyen d'un matériau présentant des ondulations dans le sens perpendiculaire respectivement à l'axe (F) du fémur et à l'axe (T) du tibia de manière à éviter le glissement longitudinal sur le membre inférieur ;
- le dispositif articulé (1,2) comporte une butée (10,11) adaptée pour limiter sa course en extension et une butée (12) adaptée pour limiter sa course en flexion ainsi qu'un moyen de blocage des deux disques entre eux : les butées et ledit moyen pouvant être du type vis et écrou ;
- les moyens de liaison (5,6) sont réalisés en matière plastique souple, moulée ou thermoformée, ou en une matière du type élastomère.

Les liaisons des bras fémoraux et tibiaux avec d'une part les disques (1,2) et d'autre part avec les moyens de liaison (5,6), peuvent être assurées par des rivets ou par des ensembles vis-écrous.

L'orthèse peut être réalisée en plusieurs tailles.
Bien entendu, l'invention n'est pas limitée aux modes de réalisation décrits et représentés pour lesquels on pourra prévoir d'autres variantes, en particulier dans :
- la nature, la conception, les dimensions et les formes des divers sous-ensembles constitutifs de l'orthèse ;
- les moyens de liaison desdits sous-ensembles entre eux ;
- les moyens de fixation de l'orthèse aux membres inférieurs du patient.

## Revendications

1. Orthèse dynamique polycentrique de l'articulation du genou du genre comportant :
a) un dispositif articulé (1,2), disposé sur au moins l'une des faces externe ou interne du genou, adapté pour reproduire la cinématique du genou lors du mouvement de flexion et d'extension du membre inférieur concerné du patient et constitué de deux flasques discaux (1) et (2), en superposition, pourvus, pour le premier, de deux points articulés distincts (1a) et (1b) et, pour le second, de deux autres points articulés distincts (2a) et (2b) ;
b) deux bras (3) et (4), reliés à chaque dispositif articulé (1,2), disposés, pour le premier, sur au moins l'une des faces externe ou interne de la cuisse (7), selon un axe parallèle à celui (F) du fémur, pour le second, sur au moins l'une des faces externe ou interne de la jambe (8), au niveau du mollet, selon un axe parallèle à celui (T) du tibia ; l'extrémité inférieure du bras fémoral (3) étant reliée à deux points articulés (1a) et (2a) appartenant respectivement aux flasques discaux (1) et (2) et l'extrémité supérieure du bras tibial (4) étant reliée aux deux autres points articulés (1 b) et (2b) appartenant respectivement auxdits flasques discaux (1) et (2) ; les quatre points articulés (1a), (1b), (2a) et (2b) étant disposés de manière à reproduire le diagramme des points de contact successifs physiologiques entre fémur et tibia ; les extrémités des bras fémoral et tibial étant placées entre lesdits deux flasques discaux (1) et (2) ;
c) deux moyens de liaison (5) et (6), adaptés pour maintenir respectivement le ou les bras fémoraux (3) sur la cuisse (7) et le ou les bras tibiaux (4) sur la jambe (8) du patient ;
**caractérisée en ce que** les quatre points articulés (1a), (1b), (2a) et (2b) sont disposés de manière à constituer, lorsque l'articulation est en extension, les sommets d'un quadrilatère déformable qui ont pour coordonnées, par rapport à des axes orthogonaux X et Y, (0,Y1) pour le premier (1a), (X2,Y1) pour le second (2a), (-X1,0) pour le troisième (2b) et (X3,Y3) pour le quatrième (1b), avec X2 supérieur à Y1, X3 supérieur à X2 et Y3 inférieur à Y1.

2. Orthèse, selon la revendication 1, **caractérisée en ce que** la valeur de Y3 est comprise entre 40 et 60% de celle de Y1, la valeur de X3 est comprise entre 140 et 160% de celle de X2 et la valeur de X1 est comprise entre 1 et 20% de celle de X2.

3. Orthèse, selon la revendication 1 ou 2, **caractérisée en ce que** le moyen de liaison (5), adapté pour maintenir le ou les bras fémoraux (3) sur les faces externe et ou interne de la cuisse (7) du patient, est constitué d'un premier élément (5a), apte à se plaquer sur la partie antérieure de la cuisse et d'un deuxième élément (5b) apte à se plaquer sur la partie postérieure de ladite cuisse.

4. Orthèse, selon la revendication 1 ou 2, **caractérisée en ce que** le moyen de liaison (6), adapté pour maintenir le ou les bras tibiaux (4) sur la jambe (8) du patient, est constitué d'un premier élément (6a) apte à se plaquer sur la partie antérieure de celle-ci et d'un deuxième élément (6b) apte à se plaquer sur sa partie postérieure, au niveau du mollet.

5. Orthèse, selon la revendication 3 ou 4, **caractérisée en ce que** chacun des éléments (5a), (5b), (6a) et (6b) constitutifs des moyens de liaison (5) et (6) est associé à une lanière (9) apte à entourer respectivement la cuisse ou le mollet et munie à ses extrémités d'un moyen de fermeture (9a) du type auto-agrippant.

6. Orthèse, selon la revendication 3 ou 4, **caractérisée en ce que** les éléments (5a), (5b), (6a) et (6b) constitutifs des moyens de liaison (5) et (6) sont réalisés au moyen d'un matériau présentant des ondulations dans le sens perpendiculaire respectivement à l'axe (F) du fémur et à l'axe (T) du tibia.

7. Orthèse, selon la revendication 1 ou 2, **caractérisée en ce que** le dispositif articulé (1,2) comporte une butée (10,11) adaptée pour limiter sa course en extension.

8. Orthèse, selon la revendication 1 ou 2, **caractérisée en ce que** le dispositif articulé (1,2) comporte une butée (12) adaptée pour limiter sa course en flexion.

9. Orthèse, selon la revendication 1 ou 2" **caractérisée en ce que** les moyens de liaison (5,6) sont réalisés en matière plastique souple, moulée ou thermoformée, ou en une matière du type élastomère.

## Claims

1. Polycentric dynamic knee joint orthesis of the type comprising:
a) a jointed device (1,2), arranged on at least one of the outer or inner faces of the knee, suitable for reproducing the kinematics of the knee during the bending and extension movement of the patient's lower limb concerned and consisting of two disc flanges (1) and (2), in a superimposed position, equipped, in the case of the first, with two separate jointed points (1a) and (1b) and, in the case of the second, two other separate jointed points (2a) and (2b);
b) two arms (3) and (4), connected to each jointed device (1,2), arranged, in the case of the first, on at least one of the outer or inner faces of the thigh (7), along an axis parallel with that (F) of the femur, in the case of the second, on at least one of the outer or inner faces of the leg (8), at the level of the calf, along an axis parallel with that (T) of the tibia; the lower end of the femoral arm (3) being connected to two jointed points (1a) and (2a) belonging to the disc flanges (1) and (2), respectively, and the upper end of the tibial arm (4) being connected to two other jointed points (1b) and (2b) belonging to said disc flanges (1) and (2), respectively; the four jointed points (1a), (1b), (2a) and (2b) being arranged so as to reproduce the diagram of the successive physiological contact points between the femur and the tibia; the ends of the femoral and tibial arms being positioned between said two disc flanges (1) and (2);
c) two connection means (5) and (6), suitable for holding the femoral arm(s) (3) on the thigh (7) and the tibial arm(s) (4) on the leg (8) of the patient, respectively;
**characterised in that** the four jointed points (1a), (1b), (2a) and (2b) are arranged so as to form, when the joint is in the extended position, the apexes of a deformable quadrilateral for which the coordinates, with respect to orthogonal X and Y axes, are (0,Y1) in the case of the first (1a), (X2,Y1) in the case of the second (2a), (-X1,0) in the case of third (2b) and (X3,Y3) in the case of the fourth (1b), where X2 is greater than Y1, X3 is greater than X2 and Y3 is less than Y1.

2. Orthesis, according to claim 1, **characterised in that** the value of Y3 is between 40 and 60% of that of Y1, the value of X3 is between 140 and 160% of that of X2 and the value of X1 is between 1 and 20% of that of X2.

3. Orthesis, according to claim 1 or 2, **characterised in that** the connection means (5), suitable for holding the femoral arm(s) (3) on the outer or inner face of the patient's thigh (7), consists of a first part (5a), capable of pressing against the anterior part of the thigh and a second part (5b) capable of pressing against the posterior part of said thigh.

4. Orthesis, according to claim 1 or 2, **characterised in that** the connection means (6), suitable for holding the tibial arm(s) (4) on the patient's leg (8), consists of a first part (6a) capable of pressing against the anterior part thereof and a second part (6b) capable of pressing against the posterior part, at the level of the calf.

5. Orthesis, according to claim 3 or 4, **characterised in that** each of the constituent parts (5a), (5b), (6a) and (6b) of the connection means (5) and (6) is associated with a strap (9) capable of encircling the thigh or the calf, respectively and equipped at the ends thereof with closing means (9a) of the self-adhesive type.

6. Orthesis, according to claim 3 or 4, **characterised in that** constituent parts (5a), (5b), (6a) and (6b) of the connection means (5) and (6) are made using a material comprising undulations in the direction perpendicular to the axis (F) of the femur and to the axis (T) of the tibia, respectively.

7. Orthesis, according to claim 1 or 2, **characterised in that** the jointed device (1,2) comprises a stop (10,11) suitable for limiting its travel in the extending position.

8. Orthesis, according to claim 1 or 2, **characterised in that** the jointed device (1,2) comprises a stop (12) suitable for limiting its travel in the bending position.

9. Orthesis, according to claim 1 or 2, **characterised in that** the connection means (5,6) are made of a flexible, moulded or thermoformed plastic material or of an elastomer type material.

## Patentansprüche

1. Polyzentrische dynamische Orthese des Kniegelenks in der Art, die umfasst:
a) eine Gelenkvorrichtung (1,2), die an mindestens einer Außen- oder Innenseiten des Knies angeordnet ist, zur Reproduktion der Kinematik des Knies bei Beuge- und Streckbewegungen der entsprechenden unteren Gliedmaße des Patienten geeignet und gebildet von zwei übereinanderliegenden scheibenförmigen Flanschen (1) und (2), wobei der erste mit zwei unterschiedlichen Gelenkpunkten (1a) und (1b) und der zweite mit zwei anderen unterschiedlichen Gelenkpunkten (2a) und (2b) ausgestattet ist;
b) zwei Arme (3) und (4), die mit jeder Gelenkvorrichtung (1,2) verbunden sind, wobei der erste auf mindestens einer der Außen- oder Innenseiten des Schenkels (7) parallel zu einer Achse (F) des Oberschenkelknochens angeordnet ist und der zweite auf mindestens einer Außen- oder Innenseite des Beins (8) auf Ebene der Wade parallel zu einer Achse (T) des Schienbeins; wobei das untere Ende des Oberschenkelknochenarms (3) mit zwei Gelenkpunkten (1a) und (2a) verbunden ist, die jeweils zu den scheibenförmigen Flanschen (1) und (2) gehören, und das obere Ende des Schienbeinarms (4) mit zwei anderen Gelenkpunkten (1b) und (2b) verbunden ist, die jeweils zu den besagten scheibenförmigen Flanschen (1) und (2) gehören; wobei die vier Gelenkpunkte (1a), (1b), (2a) und (2b) derart angeordnet sind, um das Diagramm der aufeinanderfolgenden physiologischen Kontaktpunkte zwischen Oberschenkelknochen und Schienbein zu reproduzieren; wobei die Enden des Oberschenkelknochenarms und des Schienbeinarms zwischen den besagten zwei scheibenförmigen Flanschen (1) und (2) angeordnet sind;
c) zwei Verbindungsmittel (5) und (6), zum Halten jeweils des oder der Oberschenkelknochenarme (3) auf dem Oberschenkel (7) und des oder der Schienbeinarme (4) auf dem Bein (8) des Patienten geeignet;
**dadurch gekennzeichnet, dass** die vier Gelenkpunkte (1a), (1b), (2a) und (2b) derart angeordnet sind, dass sie, wenn das Gelenk gestreckt ist, die Scheitelpunkte eines verformbaren Vierecks bilden, deren Koordinaten in Bezug zu den orthogonalen Achsen X und Y, (0,Y1) für den ersten (1a), (X2,Y1) für den zweiten (2a), (-X1,0) für den dritten (2b) und (X3,Y3) für den vierten (1b) sind, mit X2 größer als Y1, X3 größer als X2 und Y3 kleiner als Y1.

2. Orthese nach Anspruch 1, **dadurch gekennzeichnet, dass** der Wert von Y3 40 bis 60 % des Werts von Y1 beträgt, der Wert von X3 140 bis 160 % des Werts von X2 beträgt und der Wert von X1 1 bis 20 % des Werts von X2 beträgt.

3. Orthese nach Anspruch 1 oder 2, **dadurch gekennzeichnet, dass** das Verbindungsmittel (5), das geeignet ist, den oder die Oberschenkelknochenarme (3) auf den Außen- oder Innenseiten des Schenkels (7) des Patienten zu halten, von einem ersten Element (5a) gebildet wird, das an der Vorderseite des Schenkels anliegt, und von einem zweiten Element (5b), das an der Rückseite des besagten Schenkels anliegt.

4. Orthese nach Anspruch 1 oder 2, **dadurch gekennzeichnet, dass** das Verbindungsmittel (6), das geeignet ist, den oder die Schienbeinarme (4) auf dem Bein (8) des Patienten zu halten, von einem ersten Element (6a) gebildet wird, das an dessen Vorderseite anliegt, und von einem zweiten Element (6b), das an seiner Rückseite auf Ebene der Wade anliegt.

5. Orthese nach Anspruch 3 oder 4, **dadurch gekennzeichnet, dass** zu jedem der Elemente (5a), (5b), (6a) und (6b), die Bestandteil der Verbindungsmittel (5) und (6) sind, ein Riemen (9) gehört, der geeignet ist, jeweils den Oberschenkel oder die Wade zu umfassen und an seinen Enden mit einem Verschlussmittel (9a) vom Typ eines selbsthaftenden Mittels ausgestattet ist.

6. Orthese nach Anspruch 3 oder 4, **dadurch gekennzeichnet, dass** die Elemente (5a), (5b), (6a) und (6b), die Bestandteil der Verbindungsmittel (5) und (6) sind, aus einem Material ausgeführt sind, das jeweils senkrecht zur Achse (F) des Oberschenkelknochens und zur Achse (T) des Schienbeins Wellen aufweist.

7. Orthese nach Anspruch 1 oder 2, **dadurch gekennzeichnet, dass** die Gelenkvorrichtung (1,2) einen Anschlag (10,11) umfasst, der dazu geeignet ist, deren Streckung zu begrenzen.

8. Orthese nach Anspruch 1 oder 2, **dadurch gekennzeichnet, dass** die Gelenkvorrichtung (1,2) einen Anschlag (12) umfasst, der dazu geeignet ist, deren Beugung zu begrenzen.

9. Orthese nach Anspruch 1 oder 2, **dadurch gekennzeichnet, dass** die Verbindungsmittel (5,6) aus einem elastischen, geformten oder thermogeformten Kunststoffmaterial ausgeführt sind, oder aus einem Material vom Typ eines Elastomers.
